# EUROPEAN PATENT SPECIFICATION

(11) **EP 2 137 292 B1**
(45) Date of publication and mention of the grant of the patent: **22.05.2019**
(21) Application number: 08759378.6
(22) Date of filing: 17.04.2008
(51) Int. Cl.: C12H 1/14, C07K 14/39, C12P 21/00, C12P 21/06

(54) **PEPTIDE MIXTURE AS WINE STABILISER**
PEPTIDMISCHUNG ALS WEINSTABILISATOR
MÉLANGE PEPTIDIQUE POUR LA STABILISATION DES VINS

(30) Priority: 20.04.2007 EP 07106638
(43) Date of publication of application: 30.12.2009
(73) Proprietor: Rymco International AG, 6300 Zug (CH)
(72) Inventor: LANKHORST, Peter, Philip, NL-3051 JE Rotterdam (NL); BIJL, Hendrik, Louis, NL-3131 ZD Vlaardingen (NL)
(74) Representative: Curran, Clair
(86) International application number: PCT/EP2008/054690
(87) International publication number: WO 2008/128972

(56) References cited:
- EP-A- 1 094 117
- WO-A-2006/067145
- WO-A-2006/067147
- US-A- 6 139 891
- US-A1- 2003 165 592
- US-A1- 2004 101 934
- KIM S-K ET AL: "Isolation and Characterization of Antioxidative Peptides from Gelatin Hydrolysate of Alaska Pollack Skin" JOURNAL OF AGRICULTURAL AND FOOD CHEMISTRY, AMERICAN CHEMICAL SOCIETY. WASHINGTON, US, vol. 49, 1 January 2001 (2001-01-01), pages 1984-1989, XP002979282 ISSN: 0021-8561
- SEKI, T. ET AL.: "Effect of AMinated Gelatin on the Nasal Absorption of Insulin in Rats" BIOL. PHARM. BULL., vol. 28, no. 3, 2005, pages 510-514, XP002492332

## Description

### Field of the invention

The present invention relates to the use of a composition comprising at least 2.5% of a peptide mixture based on dry weight characterized in that the peptides from yeast have a molecular weight ≥3 kDa and ≤10 kDa, in the stabilisation of wines, in particular to prevent or retard the formation of tartrate salts in the wine.

### Background of the invention

The production of wine is characterised by the following steps which are well known to those skilled in the art: a) extracting wine must from grapes; b) subjecting the wine must to fermentation under the action of yeast to yield wine which is subsequently separated from solid residues; c) optionally subjecting the wine to aging and d) bottling or packaging the wine. After separation of wine from solid residues present after fermentation, other insoluble are generally formed in the wine in the subsequent steps of its production (for instance solids due to the precipitation of tartrate salts for instance when the wine undergoes cold stabilisation). Therefore wine production, especially if performed at large scale, very often comprises the step of filtering the wine to remove the above mentioned insoluble compounds formed just prior to bottling or packaging of the wine. Wine making can comprise, depending on the wine to be produced, additional steps to those mentioned above. All these steps are known to those skilled in the art.

The presence of tartaric salts, potassium hydrogen tartrate (KHT) or calcium tartrate (CaT) is one of the major causes of instability of wines. Tartaric acid is the main organic acid produced by the grape berry during its development. It is dissolved in the form of potassium and calcium salts into grape musts during the processing of berries. During the fermentation, the solubility of salts of tartaric acid decreases with the increase of ethanol concentration (due to the fermentation of sugars). In young wines, potassium hydrogen tartrate (KHT) is always present in supersaturating concentrations and crystallises spontaneously. After bottling of wines, the KHT-instability may become a commercial problem due to the unpredictable character of the crystallisation. Besides, consumers often perceive the presence of crystals in the bottle as a sign of inferior quality of the wine. Physical treatments can be used prior to bottling of the wine to prevent crystallisation of tartrate salts. These treatments consist in promoting the crystallisation by cooling the wine to -4°C or in elimination of the potassium and tartaric ions by electrodialysis or by the use of ion-exchange resins. However, these time- and energy-consuming processes are supposed to alter the colloidal equilibrium of wines.

The alternative to physical treatments of wines is to use additives, which prevent the nucleation and/or the growth of KHT crystals. In an (industrial) wine making process comprising a step for stabilising wine by addition of a wine stabiliser (for instance mannoprotein) said wine stabiliser can be added either to the wine must or to the wine. Generally it will be added to the wine, normally during ageing and prior to filtration and bottling. In case addition of the wine stabiliser occurs during aging and prior to filtration and bottling it has been found that activity of the wine stabiliser (for instance mannoprotein) as stabiliser against precipitation of tartrate salts decreases after filtration of the wine. Therefore a higher dose of wine stabiliser should be added in order to achieve the desired stabilisation in the bottled wine. A solution to this problem is therefore to add the wine stabiliser as a solution after the last filtration step which the wine undergoes prior to bottling.

Carboxymethylcellulose and meta-tartaric acid belong to the group of additives which inhibit the growth of KHT crystals. However, carboxymethylcellulose is not approved as a wine additive in current regulation. Meta-tartaric acid, on the other hand, is unstable at the pH of wine and at the temperature at which wine is stored. Over time, the meta-tartaric acid will hydrolyse and its protective effect will disappear. Therefore, its use is restricted to low quality wines for quick consumption. Another drawback is that, since an additive preferably is a natural component of wine, this is definitely not the case with meta-tartaric acid or carboxymethylcellulose. Natural additives, which are active against both nucleation and growth rate of KHT crystals, are preferred to chemical additives. An example of a natural additive is mannoprotein. Mannoprotein is, together with glucan, the main component of cell walls in yeasts (Lipke P.N. et al, J. Bacteriol. (1998) 180(15): 3735-3740). Mannoprotein is mainly obtained from yeast cells by two types of methods: physical methods and enzymatic methods. The most common physical method to obtain mannoprotein is that described in Peat S. et al, J. Chem. Soc. London (1961) 28-35, wherein mannoprotein is obtained by heat-extracting yeast cells at neutral pH. Enzymatic methods to obtain mannoprotein from yeast are e.g. described in WO 96/13571, and essentially comprise treatment of isolated yeast cell walls with a β- glucanase preparation and isolation of the product via ultrafiltration. The activity of mannoproteins extracted either by heat or by β -glucanase preparation against tartaric acid salt precipitation, has been determined. It has been found that the active fractions against tartrate stabilisation are mannoproteins extracted from yeast cell walls using β - glucanase preparation, having a molecular weight of -30-50 kDa and which are highly glycosylated (Dubourdieu D. et al, J. Int. Sci. Vigne Vin. (1997) vol. 31 (1) pp. 23-31).

Mannoprotein may have the disadvantage that, once added to wine, it gives rise to undesirable turbidity and, in some cases, to precipitation of by-products. Moreover, the effectiveness of mannoprotein to prevent nucleation and/or growth of KHT-crystals is not always satisfactory. EP-A-10941 17 describes a method to produce soluble solids of mannoprotein wherein the problem of the turbidity caused by by-products present in mannoprotein preparations is reduced. In this method, yeast cell walls are isolated after autolysis and are subsequently subjected to a selective hydrolysis either at 95-100°C for 15-30 hours or under the action of β -glucanase. In both cases mannoprotein and other impurities are solubilised. Several steps are necessary to purify the solubilised mannoprotein. Mannoprotein which is fully soluble in wine and which is very active against precipitation of salts of tartaric acid in wine has been described in WO2006/067145.

US6139891 relates to stabilisation of wine against tartrate crystallisation by adding mannoproteins from yeast. The active mannoprotein fraction has a molecular weight of 41.6 kDa.

There is however still the need for wine additives displaying an improved effect on the prevention and/or retardation of the formation of tartaric acid salts in wine and in which by-product-caused turbidity and/or by-product precipitation is very limited or absent. US-2004/0101934 discloses the use of a yeast-derived bioactive peptide having activities such as an anti-stress agent, an anti-fatigue agent, premenstrual syndrome (PMS) and menstrual pain relaxants, and a brain neurotrophic factor.

It has now been surprisingly found that peptides of a specific molecular weight are particularly effective in stabilising wine against crystallisation of salts of tartaric acid.

### Detailed description of the invention

### Definitions

A "peptide mixture" is defined herein as a mixture of peptides with different lengths with respect to the number of amino acids in the peptide as well as different composition with respect to the type of amino acids.

A "peptide" is defined herein as a compound comprising two or more amino acids residues which are linked through peptide bonds.

"Food grade component" is defined herein as a component which is safe for use in food.

"Protein hydrolysate" is defined herein as acid or enzymatically treated protein substrates containing mixtures of amino acids and peptides in varying proportions that are determined by the degree of hydrolysis and/or the type of enzymes used.

"Yeast extract" is defined herein as a composition comprising the water-soluble components extracted from yeast cells. In general, yeast extracts comprise amino acids, proteins, peptides, vitamins, carbohydrates and salts like phosphates. Yeast extracts may as well comprise 5'-ribonucleotides and/or oligonucleotides.

"Mannoprotein" is defined herein according to RESOLUTION OENO 26/2004 of the OIV (Organisation Internationale de la Vigne et du Vin) as a product which can be extracted from Saccharomyces cerevisiae cells and/or yeast cell walls by physicochemical or enzymatic methods. Mannoproteins are different structures depending on their molecular weight, their degree and type of glycosylation and their load size.

"Protease activity" is defined herein as the total proteolytic enzyme activity due to exoproteases and endoproteases.

"Free of protease activity" is defined herein as that protease activity in the liquid formulation will be lower than the minimum activity detectable with the method used to measure protease activity. Depending on the type of proteases which are expected to be present in the liquid formulation, specific methods to measure protease can be used and specific protease units can be defined. These methods are known to those skilled in the art.

"Total amount of viable microorganisms" is defined herein as the total amount of aerobic and anaerobic psychrophilic microorganisms + aerobic and anaerobic mesophilic microorganisms + aerobic and anaerobic thermophilic microorganisms present in the solution.

In a first aspect the present invention provides the use of a composition according to claim 1.

The "molecular weight" as used herein, for instance the molecular weight of the peptide or biomolecule, is defined by the experimental method to determine the molecular weight. One such a method is ultrafiltration which is very well known in the art. A molecule having a molecular ≥3 kDa means that such a molecule is retained by an ultrafiltration membrane with a cut-off of 3 kDa. A molecule having a molecular weight ≤10 kDa means that such a molecule permeates an ultrafiltration membrane with a cut-off of 10 kDa. Such membranes may be of the type of regenerated cellulose or polyethersulfone.

In a preferred embodiment the composition comprises at least 4% w/w of the peptide mixture wherein the peptides have the molecular weight as stated hereinbefore, more preferably at least 6%, more preferably at least 8%, more preferably at least 10% w/w, more preferably at least 20% w/w, more preferably at least 25% w/w, more preferably at least 30% w/w, more preferably at least 40% w/w, more preferably at least 50% w/w, more preferably at least 60% w/w, more preferably at least 70% w/w, more preferably at least 80% w/w, more preferably at least 90% w/w, more preferably at least 95% w/w, most preferably at least 99% based on dry matter of the composition.

The composition may further comprise one or more biomolecules such as carbohydrates and/or oligonucleotides depending on the source used for the manufacturing of the peptide mixture. An example of such a carbohydrate is mannan. Preferably, these biomolecules have the same molecular weight as defined above for the peptides in the composition of the invention.

The composition may be in the form of a solid or in the form of a liquid or solution. The composition in the form of a solid formulation has the advantage that such a formulation has good storage stability and is in particular microbiologically stable. A disadvantage may be that the solid dissolves only slowly and/or incompletely. This may be the case when the wine or wine must to which the composition is added, is kept at a low storage temperature, e.g. below 15°C or even lower e.g. below 10°C. Another disadvantage of the composition in the form of a solid formulation may be that it is difficult to obtain a homogenous concentration of the composition of the invention in the wine or wine must, due to for instance mixing problems.

It may therefore be preferred to add the composition to wine or wine must in the form of a liquid or solution. This may be achieved by producing the composition directly in the form of a - preferably ready-to-use - liquid formulation, preferably by the manufacturer or, alternatively, by making a solution of the solid formulation of the wine stabilising composition, preferably by the winemaker. Such a solution may be made in water or preferably in a suitable volume of the wine or wine must. Depending on the effective amount of the composition which is necessary to stabilize the wine against tartrate crystallization, the solution of the composition may be prepared by dissolving the appropriate amount of the solid formulation on the basis of this effective amount as well as a desired and practical dosage of the solution to the wine or wine must. It may for instance be preferred to dilute the solution into the wine or wine must 100-fold or 1000-fold. The effective amount of the solid formulation as well as the dosage of the solution may easily be determined by the skilled person without undue burden and may for each wine be different since each wine will have its own tartrate crystallization behaviour.

In a preferred example, the composition produced directly in the form of a, preferably ready-to-use, liquid formulation or, alternatively, made as a solution from the solid formulation as described hereinbefore, has a dry matter content between 50 - 500 g/l, more preferably between 100 - 400 g/l and most preferably between 200 - 300 g/l. The dry matter content of the liquid formulation or the solution may be combined with any content of the peptide mixture as disclosed hereinbefore. For example, if the solution has a dry matter content between 50 - 500 g/l and 2.5% peptide mixture (based on the total dry matter), this means the concentration of the peptide mixture in this solution is between 1.25 and 12.5 g/l (w/v). It will be understood by the skilled person that in the liquid formulation or the solution of the composition all preferred ranges for the dry matter content (in g/l) may be combined with all preferred ranges for the content of the peptide mixture expressed as percentage of the total dry matter of the composition.

A highly preferred ready-to-use liquid composition has a dry weight content of approximately 200 g/l and a peptide mixture content of 10% based on dry weight which is equal to approximately 20 g/l of peptides.

The liquid formulation or the solution of the composition as described hereinbefore finds its preferred use in the stabilisation of wine or must. For this reason it is important that the solution is devoid of the presence of any harmful microorganism. Furthermore the presence of any viable microorganism can have an impact on the storage stability of the solution and in the preservation of its activity as wine stabiliser. For these reasons in one example, the solution has a total amount of viable microorganisms, measured in Colony Forming Unit (CFU) / ml which is lower than 10, preferably lower than 5. More preferably "the solution" is sterile. The total amount of viable microorganisms present in the solution as described hereinbefore, measured in Colony Forming Unit (CFU) / ml is defined in Materials and Methods and is established by determination of the total plate count (TPC), a method which is well known to those skilled in the art and shortly described in Materials and Methods.

The liquid formulation or the solution of the composition as described hereinbefore is preferably packed in a sterile container which may be hermetically closed during transport and storage.

The liquid formulation or the solution of the composition as described hereinbefore is preferably an aqueous solution.

The pH of the liquid formulation or the solution of the composition as described hereinbefore is preferably such that the stability of the composition is preserved in time and such as to avoid turbidity of the solution. Therefore the pH of the solution is preferably comprised between 3 and 8, more preferably between 4 and 7, most preferably between 5 and 6.

The liquid formulation or the solution of the composition as described hereinbefore is preferably microbiologically stable in time. Therefore in a preferred example at least a stabilising additive is added to the solution. More preferably the stabilising additive is sulfur dioxide. More preferably sulfur dioxide can be added to the solution in the form of bisulfite salt, more preferably as sodium or potassium bisulfite (K₂S₂O5). Addition of sulphur dioxide has another advantage next to maintain the mannoprotein solution microbiologically stable. Mannoprotein solutions have a colour which can vary from light yellow to brownish. However the use of a brownish solution for the stabilisation of wine would not be very appealing to the winemaker. Therefore a mannoprotein solution to be used in wine or must should preferably be only slightly coloured. It has been found that the addition of sulphur dioxide to a mannoprotein solution can reduce the colour thereof from brownish to pale yellow. Therefore the amount of sulphur dioxide to be added to the solution is preferably in the range of 1 to 20 g/l (measured as amount of K₂S₂O₅ added).

The composition may comprise one or more additional components preferably food grade. The component is preferably suitable for use in wine, as defined above. Examples of suitable components are protein substrates, protein hydrolysates, yeast extracts, carboxy methyl cellulose, wine additives or mixtures of two or more of these components. Examples of wine additives are meta-tartrate, Arabic gum, or mannoprotein. A specially preferred wine additive is mannoprotein. Another specially preferred component is a protein hydrolysate. Typical protein substrates for the preparation of protein hydrolysates are vegetable proteins such as wheat gluten, corn gluten, soy protein, rape seed protein, pea protein, alfalfa protein, sunflower protein, fabaceous bean protein, cotton or sesame seed protein, maize protein, barley protein, sorghum protein, potato protein, rice protein, coffee proteins. Other possible protein substrates are animal proteins such as milk protein (for instance casein or whey protein), egg white, fish protein, meat protein including gelatin, collagen, blood protein (for instance haemoglobin), hair, feathers and fish meal.

When the composition comprises protein hydrolysate, this is preferably selected from a protein hydrolysate of animal, vegetable or microbial origin, more preferably selected from casein hydrolysate, egg albumin hydrolysate, gelatin hydrolysate, bovine serum albumine hydrolysate, lysozyme hydrolysate, wheat protein hydrolysate, soy protein hydrolysate, pea protein hydrolysate or a mixture of two or more of these components.

Preferably, the liquid formulation or the solution of the composition as described hereinbefore is translucent at the concentrations mentioned. The translucency may be quantified as the reciprocal of the turbidity of the solution at a concentration of 200 g/l of total dry matter measured with nephelometry according to standard methods. The liquid formulation or the solution of the composition preferably has, at a dry matter content of 200 g/l, a turbidity:
- at pH 4: ≤500 NTU, preferably ≤250 NTU, more preferably ≤200 NTU, more preferably ≤150 NTU, more preferably ≤100 NTU, more preferably ≤50 NTU, most preferably ≤35 NTU; and/or
- at pH 8.0: ≤250 NTU, preferably ≤200 NTU, more preferably ≤150 NTU, more preferably ≤100 NTU, more preferably ≤50 NTU, more preferably ≤25 NTU, more preferably ≤20, most preferably ≤15 NTU

The composition, when dissolved in wine or wine must, should give a translucent and stable wine. Therefore in a preferred example the composition of the invention when dissolved in water to yield a solution with a dry matter content of a 1 g/l has a turbidity of ≤4 NTU, preferably of ≤2 NTU, more preferably of ≤1 NTU and/or when dissolved in 12% ethanol in water (v/v) to yield a solution with a dry matter content of a 1 g/l and at a pH of 3.5 has a turbidity of ≤20 NTU, preferably of ≤10 NTU, even more preferably of ≤5 NTU, most preferably of ≤1 NTU, and/or when dissolved in white wine to yield a solution with a dry matter content of a 0.2 g/l has a turbidity of ≤10 NTU, preferably of ≤5 NTU, even more preferably of ≤2 NTU, most preferably of ≤1 NTU.

The composition for use according to the first aspect of the invention may be prepared according to several methods. For example the peptide mixture may be produced by using liquid phase or solid phase peptide synthesis or it may be prepared by hydrolysis of a peptide source. Preferably the composition of the first aspect is prepared by enzymatic hydrolysis of a peptide source, preferably a peptide source from natural sources such as protein substrates, protein hydrolysates, yeast extracts etceteras.

A process to prepare the composition for use according the first aspect of the invention comprises the step of hydrolyzing a peptide source comprising ≤2.5% of the peptide mixture with the molecular weights of the peptides as defined hereinbefore.

The peptide source may be hydrolysed by controlled proteolysis by choosing a suitable enzyme and conditions of pH, temperature and time, aiming at the formation of peptides with molecular weights ≥3 kDa and ≤10 kDa. Hydrolysis can be performed using chemical methods known to those skilled in the art (e.g. as described in "Savory Flavours", 1995, by T.W. Nagodawithana, Esteekay Associates Inc., Wisconsin, USA, pages 233-237), or enzymatic methods. Preferably hydrolysis is performed by enzymatic methods. Enzymatic methods to hydrolyse peptide sources are also known to those skilled in the art (Adler-Nissen, J (1986) Enzymic hydrolysis of food proteins, Elsevier Appl. Sci. Publ.).

Suitable protein substrates that may serve as peptide source are casein, egg albumin, gelatine, bovine serine albumin, lysozyme, wheat protein, soy protein, pea protein, yeast, or a mixture of two or more of these components. Preferably, the protein substrate is yeast.

Suitable protein hydrolysates that may serve as peptide source are casein hydrolysate, egg albumin hydrolysate, gelatine hydrolysate, bovine serine albumine hydrolysate, lysozyme hydrolysate, wheat protein hydrolysate, soy protein hydrolysate, pea protein hydrolysate, yeast extract, or a mixture of two or more of these components.

After hydrolysis of the peptide source as described hereinbefore said peptide source may comprise less than 2.5% based on total dry weight of a peptide mixture characterized in that the peptides have a molecular weight between 3 kDa and 10 kDa. In order to increase the concentration of said peptide mixture to at least said peptide mixture 2.5% enriched. Enrichment may be achieved using separation techniques known in the art such as HPLC, size exclusion chromatography, Gel Permeation Chromatography, selective precipitation.

A preferred separation technique to enrich the peptide mixture is membrane filtration, preferably ultrafiltration or diafiltration, under conditions suitable to increase the amount of the peptide mixture. The skilled man knows how to perform membrane filtration, preferably diafiltration or ultrafiltration, under conditions suitable to yield a composition comprising at least 2.5% w/w based on total dry matter of a peptide mixture with molecular weights between 3 and 10 kDa. Membrane filtration techniques, in particular ultrafiltration and diafiltration, are known to those skilled in the art. Suitable ultrafiltration or diafiltration membranes which are used in the process of the third aspect are polyethersulfone (PES) membranes, hydrophilic polyethersulfone membranes (PES H), regenerated cellulose membranes or ceramic membranes of suitable cut-off.

In an embodiment, enrichment of the peptide mixture is performed by subjecting the hydrolyzed peptide source to an ultrafiltration step using a membrane with a cut-off of 3 kDa, collecting the resulting retentate, and optionally subjecting said retentate to a second ultrafiltration step using a membrane with a cut-off of 10 kDa and collecting the resulting permeate. Alternatively the hydrolyzed peptide source is subjected to an ultrafiltration step using a membrane with a cut-off of 10 kDa, collecting the resulting permeate, optionally subjecting said permeate to a second ultrafiltration step using a membrane with a cut-off of 3 kDa and collecting the resulting retentate.

After hydrolysis of the peptide source and/or enrichment of the peptide mixture, said peptide mixture may be concentrated and optionally dried according to standard techniques, such as freeze drying or spray drying. In case the peptide mixture is collected as a liquid, the process may comprise a last sterile fitration step aimed at producing a sterile solution which can be packaged as such.

Proteases are enzymes which can be used in the production of peptide mixtures fractions or compositions thereof starting from natural sources such as peptide sources and which may be present in the peptide mixtures fraction or compositions thereof as an impurity. The presence of these impurities does not represent a problem when the peptide mixture or compositions thereof are isolated as a solid. The presence of small traces of protease activity in a solution instead might cause degradation of the peptide mixture or compositions thereof and cause a decrease of activity of the peptide mixture or of the compositions thereof in respect of stabilisation of wine towards tartrate precipitation. To produce a composition for use according to the first aspect which is free from protease activity, the process may comprise subjecting a solution of the composition to a treatment suitable to inactivate protease, preferably by subjecting a solution of the composition according to the first aspect to heat treatment at a temperature of 70°C or higher, preferably at a temperature comprised between 80°C and 140°C, wherein the heat treatment is preferably performed for a time comprised between 2 seconds and 60 minutes.

Any treatment suitable to inactivate protease activity can be used at this regard. More preferably a heating step is used to inactivate enzymatic activity. The temperature used in the heat treatment is preferably of 70°C or higher, preferably the temperature is between 80°C and 140°C. The duration of the heat treatment will be such that preferably all enzyme activity will be removed. The duration of the heat treatment will depend on the temperature used. For example lower temperatures will require a longer duration of the heat treatment while higher temperatures will require only a short time. For example, a treatment at 80°C for 30 minutes as well as a treatment at 130°C for 7 seconds are envisaged. The heat treatment is preferably performed by using a UHT treatment using conditions which are commonly used in the food or beverage industry. A UHT treatment may typically be effected at a temperature of 130-145°C for a duration of time of 2-10 seconds. With a heating treatment performed at 130°C or higher for sufficient time, for example a heating treatment at 130°C for 7 seconds, a solution will be obtained which is free from protease activity, and in general free of any enzymatic activity.

By using a heating step generally at least part of micro-organisms present in a solution of the composition according to the first aspect will be deactivated or killed. Preferably the heating treatment is a UHT treatment as indicated above because in the latter case all microorganisms or microbial spores which might be present in the solution will be killed or deactivated.

The process may comprise subjecting the composition to sterilisation, preferably to sterile filtration in case of a solution in order to remove all microorganisms. The latter can be achieved by using specific filters known to those skilled in the art. Preferably sterile filtration is performed after protease inactivation, more preferably it is performed as a last step prior to packaging.

At the end of the production process the composition can be aseptically packed according to methods known to those skilled in the art. The sterile containers which can be used can be produced of several materials. Suitable containers may be sterile bags within a cardboard box. The bags may be provided with a gland which can be perforated by a screw-on tap.

It will be understood by those skilled in the art that the process can further comprise a step in which a solution of the composition is dried by methods known to those skilled in the art, e.g. by lyophilisation or spray drying, to yield a composition in solid form, e.g. in form of a powder or granulate.

The composition for use according the first aspect can be used in any type of wine, such as for example white, rose, sparkling, and red wines. Preferably the composition is used in white wines and rose wines.

In particular, in a second aspect the invention provides a process to stabilise wine by preventing and/or retarding the crystallisation of salts of tartaric acid according to claim 2. The composition is preferably added to the wine during ageing, i.e. after fermentation but before bottling. The invention is extremely suitable for white wines and rose wines, but also for red wines or sparkling wines.

The composition is added in an effective amount to achieve a stabilizing effect. Generally, the composition can be added to wine or grape must to be used in fermentation for the production of wine in a concentration between 10-1000 mg per litre of wine or must. Good results are already obtained by adding the composition up to a final concentration in the wine of 10 to 400 mg per litre of wine. In a preferred embodiment the composition is added to wine or grape must to be used in fermentation for the production of wine in a concentration between 10-300 mg per litre, more preferably between 20 and 250 mg per litre, even more preferably between 25 and 200 mg per litre, most preferable between 30 and 150 mg per litre. The skilled person will understand that the amount to be added will also depend on the type of wine, on the addition or presence of for instance other wine stabilisers, as well as on the degree of supersaturation of the KHT in the wine prior to addition

Generally, wine unstable in respect of crystallisation of tartaric acid salts has a Tcrys that can vary between 0.5 and 6 days. Stabilized wine is obtainable by adding to wine the composition in a concentration suitable to prevent and/or retard the crystallization of salts of tartaric acid. Preferably, said wine comprises 10-1000 mg of the composition as per litre of wine. More preferably, the wine comprises 10 to 400 mg of composition per litre of wine, more preferably 10-300 mg per litre, more preferably between 20 and 250 mg per litre, even more preferably between 25 and 200 mg per litre, most preferable between 30 and 150 mg per litre. Said stabilized wine is characterized by a Tcrysstabilized wine/Tcrysunstable wine of at least 2, preferably at least 5, more preferably at least 10, even more preferably between 10 and 40 as measured according to method 1.

The solution added to the wine after filtration can be prepared just prior to adding to the wine by mixing the composition to water or wine or it can be a (ready-to-use) solution of the composition, as described in detail above. Therefore the solution may be free of protease activity and/or sterile and/or it can further comprise a stabilizer as described above.

Addition to the wine can occur either by mixing the liquid formulation with the wine prior to bottling or packaging or it can directly be added in the bottle (or package) prior to filling thereof with wine.

The invention will now be illustrated by the following examples which do not intend however to be limiting.

### EXAMPLES

### Materials and methods

### Nucleation and crystal growth of KHT

The nucleation and crystal growth of KHT in wine can be measured and quantified by the following methods (Moutounet et al. In : Actualites Œnologiques 1999 Vieme Symposium International d'Oenologie de Bordeaux (Lonvaud-Funel ed.)).

### Method 1

The first method, indicative of crystal nucleation, measures the time of appearance of crystals in the wine when stored at -4°C. A visual inspection is performed daily and the time necessary to detect the appearance of crystals (Tcrys) is expressed in number of days.

### Method 2

The second method, indicative of crystal growth, measures the Degree of Tartaric Instability (DTI) of the wine. Hereto, wines are stirred at -4°C and the initial conductivity is measured. Subsequently, calibrated crystals of KHT are added and the conductivity is then measured after a stable value has been reached. The DTI is defined as the percentage decrease of the initial conductivity.

### Method 3

The third method measures the true, dissolved tartaric acid concentration. An accurate volume of the wine is transferred into a glass vial, and mixed with the same accurate volume of D₂O containing a precisely known concentration of maleic acid. The 1H NMR spectrum is run with conditions of full relaxation, and the integral of the internal standard (maleic acid) is compared with the integral of tartaric acid. In this way the dissolved tartaric acid concentration can be determined with very high precision and accuracy.

### Amino acid determination in the peptide mixture

A precisely weighed sample of the peptide mixture was hydrolysed using 6N HCI at 110°C for 24 hrs. After standard workup to remove HCI, the hydrolysed sample was dissolved in dilute acid and precipitates were removed by centrifugation in an Eppendorf centrifuge. Amino acid analysis was carried out on the clear supernatant according to the PicoTag method as specified in the operator's manual of the Amino Acid Analysis System of Waters (Milford MA, USA). To that end a suitable sample was obtained from the liquid, added to dilute acid and homogenized. From the latter solution a new sample was taken, dried and derivatised using phenylisothiocyanate. The various derivatised amino acids present were quantitated using HPLC methods and added up to calculate the total level of amino acids in the weighed sample.

### Carbohydrate determination

The amount of carbohydrate was determined according to the well-known anthrone colorimetric method.

### Protein determination

Protein was determined using the well-known Kjeldahl method

### Turbidity determination

Turbidity was determined with a HACH 2100 N turbidity meter (Hach-Lange, Düsseldorf, Germany).

### Total plate count

Three samples of liquid formulation were stored under the specific conditions mentioned below prior to analysis and analyzed using the total plate count (TPC) method for the possible presence of psychrophilic, mesophilic, and thermophilic microorganisms capable to grow in aerobic and anaerobic atmosphere. The determination of TPC was done by pouring about 15 ml of PCA agar (Plate Count Agar, Oxoid, UK) on 1 ml of liquid formulation present in a Petri dish. The TPC incubation was executed under one of the 6 conditions mentioned below. For each condition the analysis was done in duplicate. In total for each liquid formulation sample 12 Petri dishes were prepared, 6 were incubated aerobically the other 6 anaerobically.

### Conditions for determination of aerobic and anaerobic psychrophiles:

Samples storage: At 8°C for 30 days
Analysis: TPC on PCA plates
Incubation: Aerobic or anaerobic at 8°C for 7 days

### Conditions for determination of aerobic and anaerobic mesophiles:

Storage samples: At 30°C for 10 days
Analysis: TPC on PCA plates
Incubation: Aerobic or anaerobic at 30°C for 3 days

### Conditions for determination of aerobic and anaerobic thermophiles:

Storage samples: At 55°C for 10 days
Analysis: TPC on PCA plates
Incubation: Aerobic or anaerobic at 55°C for 3 days

After the proper incubation time, each Petri dish was analysed to determine the amount of colonies formed in each dish. The amount of colonies in each dish gives the Colony Forming Unit (CFU) per millilitre of liquid formulation for the specific type of microorganism. The total amount of viable microorganism measured as CFU per millilitre of liquid formulation as defined in this patent application is given by CFU/ml (anaerobic psychrofiles) + CFU/ml (aerobic psychrofiles) + CFU/ml (anaerobic mesophiles) + CFU/ml (aerobic mesophiles) + CFU/ml (anaerobic thermophiles) + CFU/ml (aerobic thermophiles).

### Measurement of protease activity

Free amino groups that are liberated after the action of a protease on N,N-dimethylcasein react with trinitrobenzenesulphonic acid yielding a yellow colour that can be measured spectrophotometrically at 405 nm. An enzyme standard containing alkaline protease was used for calibration. Five standard solutions were made with activities from 10 - 60 U/ml. (One U is defined as the amount of enzyme needed to liberate 7.59 µmol of p-nitroanilin per minute from succinyl-L-alanyl-L-alanyl-L-propyl-L-phenylalanine-para-nitroanaline (2.18 mg/ml) at pH 8.5 (0.1 M TRIS) and 37°C). Two ml N,N-dimethylcasein solution (1.68 g/l dissolved in borax buffer containing 10 g/l disodiumtetraborate adjusted with 8.5% phosphoric acid to pH 8.5) was mixed with 200 µl standard- or sample solution and incubated for 15 minutes at 37°C. Subsequently 1.2 ml trinitrobenzenesulphonic acid solution (4 mM in 12 mM HCI) was added. After 5 minutes of incubation at 37°C the absorbance was measured at 405 nm. By using a calibration line, the activity of unknown samples could be calculated. The quantification limit of the test in a mannoprotein matrix is 600 U/ml.

### Peptide mixture characterization

The peptide mixture in the composition can for instance be characterised by amino acid analysis or NMR. The amount of peptide in the composition can be determined by the PicoTag method as indicated in material and methods.

### Determination of the amount of the peptide mixture

The amount of the peptide mixture in the composition is measured as follows. The composition is subjected to ultrafiltation. The peptide mixture which is retained by an ultrafiltration membrane with a cut-off of 3 kDa, and permeate an ultrafiltration membrane with a cut-off of 10 kDa is collected, dried, weighed and its weight percentage in respect of the amount of dry composition which underwent ultrafiltration is determined.

### Total dry weight

Total dry weight was determined by freeze-drying and subsequent weighing.

### Example 1

One gram of Bovine serum albumine (BSA) was dissolved in 50 ml of tap water. The pH was adjusted to 8. After addition of Alcalase® (Sigma Aldrich, containing protease from *Bacillus licheniformis*) the BSA solution was incubated at 60 °C for 4 hours. The pH was readjusted to pH 8 three times, and additional Alcalase was added each time. After 4 hours no pH drop was observed anymore, indicating that hydrolysis of protein had stopped. The solution was fractionated by means of ultrafiltration (UF), first on a 3 kDa YM-3 regenerated cellulose membrane (Amicon, Millipore, USA) in a stirred cell. Next, the retentate was fractionated on a 10 kDa PM-10 polyethersulfon membrane (PES) (Amicon, Millipore, USA) yielding a retentate and a permeate. The permeate of the first ultrafiltration and the retentate and permeate of the second ultrafiltration were freeze-dried, and the total dry weight of the UF fractions relative to the initial BSA hydrolysate was calculated. The dry weight percentage of the several peptide fractions is given in table 1. Most of the BSA was hydrolyzed by Alcalase® in fragments smaller than 3kDa. However, a significant part was retained on the 3kDa membrane, and a very small part was also retained by a 10kDa membrane. The UF fractions and the BSA hydrolysate were tested in white wine supersaturated in KHT (concentration of tartaric acid 26% above saturation). Solutions of 10 mg/ml were prepared and aliquots were added to wine in order to achieve 50, 100 and 200 mg/l of peptides or of BSA hydrolysate. The formation of crystals was monitored visually on a daily basis. Results are shown in table 2.

**Table 1: Total dry weight of UF fractions (UF) isolated from BSA hydrolysate (% w/w based on dry matter of BSA hydrolysate)**

| | MW >10 kDa | 3kDa<MW<10kDa | MW<3kDa |
|---|---|---|---|
| Yield (%) | 1.5 | 10 | 88.5 |

**Table 2: Activity of BSA hydrolysate and of UF fractions after enzymatic hydrolysis. First day of crystallization at -4 °C.**

| | 1 | 2 | 3 | 4 |
|---|---|---|---|---|
| concentration (mg/l) | Hydrolyzed BSA before fractionation | UF fraction (MW>10kDa) | UF fraction (3kDa<MW<10kDa) | UF fraction (MW<3kDa) |
| + 0 mg/l | 1 | 1 | 1 | 1 |
| +50 mg/l | 4 | precipitate | 8 | 2 |
| +100 mg/l | 7 | precipitate | 13 | 4 |
| +200 mg/l | 11 | precipitate | 22 | 5 |

Table 2 shows that the hydrolyzed BSA is active as an inhibitor of KHT crystallization in wine. The UF fraction retained by the 10 kDa ultrafiltration membrane (MW>10kDa) is not fully soluble in wine, causing precipitates and cloudiness in wine. The UF fraction that permeates the 3kDa regenerated cellulose membrane (MW<3kDa) is low in activity. The UF fraction retained on a 3kDa regenerated cellulose membrane, but permeating through the 10 kDa polyethersulfon membrane (3kDa< MW<10kDa) is the most active fraction.

### Example 2

Mannoproteins were recovered from yeast extract (Maxarome®, DSM Food Specialties) by means of ultrafiltration (UF) and subsequent diafiltration (4 - 10 fold) over a 4 kDa Nadir® polyethersulfone hydrophilic membrane (Microdyn-Nadir, Germany). Two sets of independent isolates were prepared (A and B). For each set of isolates, one gram of the recovered mannoproteins was dissolved in 25 ml water. The solutions were ultrafiltrated on a 10 kDa PM-10 PES membrane (Amicon, Millipore, USA) in a stirred cell. The retentates were freeze-dried and weighed yielding fractions with molecular weight >10kDa. The permeates were ultrafiltrated on a 3 kDa YM-3 regenerated cellulose membrane (Amicon, Millipore, USA) in a stirred cell. The retentates and the permeates were freeze-dried and weighed yielding UF fractions with 3kDa<MW<10kDa. The yield the UF fractions is reported in table 3.

The UF fractions were characterised for peptide and carbohydrate content as indicated in material and methods and the results are reported in table 4.

Activity of the UF fractions and of the starting mannoproteins were tested in three different wines as indicated above and the results are reported in table 5.

**Table 3: Total dry weight (% w/w) of UF fractions isolated from mannoproteins**

| | Mannoprotein > 10 kDa | 3kDa < mannoprotein < 10 kDa | mannoprotein < 3 kDa |
|---|---|---|---|
| Yield | | | |
| Isolate A | 88 | 10 | 2 |
| Isolate B | 58 | 33 | 5 |

**Table 4: Composition of mannoprotein before ultrafiltration and of the UF fractions**

| | Mannoprotein before fractionation | | Mannoprotein UF fraction (MW > 10kDa | | Mannoprotein UF fraction (3 kDa MW > 10kDa | | Mannoprotein UF fraction (MW < 3kDa | |
|---|---|---|---|---|---|---|---|---|
| Isolate | A | B | A | B | A | B | A | B |
| Carbohydrate content (% w/w) | 74 | ND | 79 | 87 | 30 | 51 | 5 | 34 |
| Peptide content (% w/w) | 26 | ND | 21 | 13 | 70 | 49 | 95 | 66 |

**Table 5: Activity of mannoprotein UF fractions. First day of crystallization at -4°C.**

| | Mannoprotein before fractionation | | | Mannoprotein UF fraction (MW > 10kDa | | | Mannoprotein UF fraction (3 kDa MW > 10kDa | | | Mannoprotein UF fraction (MW < 3kDa | | |
|---|---|---|---|---|---|---|---|---|---|---|---|---|
| Isolate | A | B | B | A | B | B | A | B | B | A | B | B |
| Wine | French Chardonnay | French rose | German white | French Chardonnay | French rose | German white | French Chardonnay | French rosé | German white | French Chardonnay | French rose | German white |

| Concentration added to wine (mg/ml) | | | | | | | | | | | | |
|---|---|---|---|---|---|---|---|---|---|---|---|---|
| + 0 mg/ml | 1 | 1 | 1 | 1 | 1 | 1 | 1 | 1 | 1 | 1 | 1 | 1 |
| + 50 mg/ml | 3 | 5 | 17 | 3 | 1 | 2 | 10 | 6 | >6 | 3 | 1 | 2 |
| + 100 mg/ml | 11 | 7 | 17 | 7 | 1 | 2 | 25 | 14 | >6 | 4 | 1 | 2 |
| + 150 mg/ml | ND | 10 | ND | ND | 1 | 2 | ND | > 27 | >6 | ND | 2 | 2 |
| + 200 mg/ml | 17 | 13 | 22 | 13 | 3 | 4 | > 25 | > 27 | >6 | 7 | 3 | 4 |

It follows from Table 5 that the most active mannoprotein UF fraction is retained by the 3kDa regenerated cellulose membrane and permeates the 10 kDa PES membrane. This mannoprotein UF fraction is strongly enriched in peptides. It also follows that the activity of the peptide mixture between 3 and 10 kDa can be different for each wine.

### Example 3

In two separate, identical experiments (A and B), 2 grams of commercially available mannoproteins (Mannostab®, Laffort, France) were dissolved in 100 ml of water. The solutions were ultrafiltrated (UF) on a 10 kDa PM-10 PES membrane (Amicon, Millipore, USA) in a stirred cell. The retentates were freeze-dried and weighed. The permeates were ultrafiltrated on a 3 kDa regenerated cellulose membrane (Amicon YM-3) in a stirred cell. The retentates and the permeates were freeze-dried and weighed. Activity in wine was tested as described above for two wines: mannoprotein from experiment A was tested using French Chardonnay; mannoprotein from experiment B was tested using German white wine. The yields of UF fractions isolated from mannoproteins is reported in table 6a (experiment A) and table 6b (experiment B). The UF fractions were characterised for peptide and carbohydrate content as indicated in material and methods and the results are reported in table 7a and 7b. Activity of the UF fractions and of the starting mannoproteins was tested in wine as indicated above and the results are also reported in table 7a and table 7b.

**Table 6a: Total dry weight of UF fractions isolated from mannoproteins (% w/w) (experiment A)**

| | UF >10 kDa | UF fraction > 3 kDa; | UF fraction <3 kDa |
|---|---|---|---|
| | | UF fraction < 10 kDa | |
| Yield | 91 | 3 | 6 |

**Table 6b: Total dry weight of UF fractions isolated from mannoproteins from (% w/w) (experiment B)**

| | UF >10 kDa | UF fraction > 3 kDa; UF fraction < 10 kDa | UF fraction <3 kDa |
|---|---|---|---|
| Yield | 76 | 10 | 6 |

**Table 7a: Activity of mannoprotein UF fractions (experiment A). First day of crystallization at -4 °C. Activity measured in French Chardonnay.**

| | 1 | 2 | 3 | 4 |
|---|---|---|---|---|
| | Commercial mannoprotein before fractionation | Commercial mannoprotein UF fraction (MW>10kDa) | Commercial mannoprotein UF fraction (3kDa<MW< 10kDa) | Commercial mannoprotein UF fraction (MW<3kDa) |
| Carbohydrate content (% w/w) | 96 | Not determined | 71 | Not determined |
| Peptide content (% w/w) | 4 | Not determined | 29 | Not determined |

| concentration mannoprotein added to wine (mg/l) | | | | |
|---|---|---|---|---|
| + 0 mg/l | 1 | 1 | 1 | 1 |
| +50 mg/l | 1 | 1 | 4 | 1 |
| +100 mg/l | 1 | 1 | 4 | 1 |
| +200 mg/l | 1 | 1 | 5 | 2 |
| +400 mg/l | Not tested | 4 | 11 | 4 |

**Table 7b: Activity of mannoprotein UF fractions (experiment B). First day of crystallization at -4 °C. Activity measured in German white wine.**

| | 1 | 2 | 3 | 4 |
|---|---|---|---|---|
| | Commercial mannoprotein before fractionation | Commercial mannoprotein UF fraction (MW>10kDa) | Commercial mannoprotein UF fraction (3kDa<MW< 10kDa) | Commercial mannoprotein UF fraction (MW<3kDa) |
| Carbohydrate content (% w/w) | 96 | 95 | 78 | 71 |
| Peptide content (% w/w) | 4 | 5 | 22 | 29 |

| concentration mannoprotein added to wine (mg/l) | | | | |
|---|---|---|---|---|
| + 0 mg/l | 1 | 1 | 1 | 1 |
| +50 mg/l | 1 | 1 | 1 | 1 |
| +100 mg/l | 2 | 1 | 3 | 1 |
| +200 mg/l | 4 | 1 | 4 | 2 |
| +300 mg/l | ND | 1 | >6 | 3 |

It follows from table 7a and table 7b that the mannoprotein UF fraction retained by a 3kDa regenerated cellulose membrane but not by the 10 kDa PES membrane is enriched in peptides, and this UF fraction is more active than the other UF fractions.

### Example 4

One gram of Lysozyme (Delvozyme, DSM) was dissolved in 50 ml of tap water. The pH was adjusted to 8. After addition of Alcalase® (Sigma Aldrich) the lysozyme solution was incubated at 60 °C for 5. The pH was readjusted to pH 8 every hour. After 5 hours no pH drop was observed, indicating that hydrolysis of protein had stopped.

The solution was fractionated by means of ultrafiltration (UF), first on a 10 kDa PM-10 polyethersulfon (PES) membrane (Amicon, Millipore, USA) in a stirred cell. Next, the permeate was fractionated on a 3 kDa YM-3 regenerated cellulose membrane (Amicon, Millipore, USA), yielding a retentate and a permeate. The retentate of the first ultrafiltration and the retentate and permeate of the second ultrafiltration were freeze-dried, and the total dry weight of the 3 UF fractions relative to the initial lysozyme starting material was calculated. The weight percentage of the several UF fractions is given in table 8. Most of the lysozyme was hydrolyzed by Alcalase® in fragments smaller than 3kDa. However, a significant part was retained on the 3kDa membrane, and a very small part was also retained by a 10kDa membrane. The UF fractions from lysozyme were tested in rose wine supersaturated in KHT. Solutions of 10 mg/ml were prepared and aliquots were added to wine in order to achieve 50, 100 and 200 mg/l of peptides. The formation of crystals was monitored visually on a daily basis. Results are shown in table 9.

**Table 8: Total dry weight of UF fractions isolated from lysozyme hydrolysate (% w/w based on dry matter of lysozyme hydrolysate)**

| | UF >10 kDa | 3kDa<UF<10kDa | UF<3kDa |
|---|---|---|---|
| Yield | 1 | 17 | 82 |

**Table 9: Activity of lysozyme derived UF fractions after enzymatic hydrolysis. First day of crystallization at -4 °C.**

| | 1 | 2 | 3 | 4 |
|---|---|---|---|---|
| concentration added to wine (mg/l) | Lysozyme before enzymatic hydrolysis | UF fraction (MW>10kDa) | UF fraction (3kDa<MW<10kDa) | UF fraction (MW<3kDa) |
| + 0 mg/l | 1 | 1 | 1 | 1 |
| +50 mg/l | Precipitate | precipitate | 11 | 3 |
| +100 mg/l | Precipitate | precipitate | >14 | 4 |
| +200 mg/l | Precipitate | precipitate | >14 | 6 |

Table 9 shows that the UF fraction retained by the 10 kDa ultrafiltration membrane (MW>10kDa) is not fully soluble in wine, causing precipitates and cloudiness in wine. The UF fraction that permeates the 3kDa regenerated cellulose membrane (MW<3kDa) is low in activity. The UF fraction retained on a 3kDa regenerated cellulose membrane, but permeating through the 10 kDa polyethersulfon membrane (3kDa<MW<10kDa) is the most active fraction.

### Example 5

One gram of Pepton (hydrolyzed protein from a vegetable source, Sigma) was dissolved in 25 ml of tap water. The pepton was used as such, without Alcalase® treatment. The solution was fractionated by means of ultrafiltration (UF), first on a 10 kDa PM-10 polyethersulfon (PES) membrane (Amicon, Millipore, USA) in a stirred cell. Next, the permeate was fractionated on a 3 kDa YM-3 regenerated cellulose membrane (Amicon, Millipore, USA), yielding a retentate and a permeate.

The retentate of the first ultrafiltration and the retentate and permeate of the second ultrafiltration were freeze-dried, and the total dry weight of the 3 UF fractions relative to the initial pepton starting material was calculated. The weight percentage of the several peptide fractions is given in table 10.

**Table 10: Total dry weight of UF fractions isolated from vegetable pepton (% w/w based on dry matter of starting material)**

| | MW >10 kDa | 3kDa<MW<10kDa | MW<3kDa |
|---|---|---|---|
| Yield | 5 | 6 | 89 |

The UF fractions from pepton were tested in white wine supersaturated in KHT. Solutions of 10 mg/ml were prepared and aliquots were added to wine in order to achieve 50, 100 and 200 mg/l of peptides. The formation of crystals was monitored visually on a daily basis. Results are shown in table 11.

**Table 11: Activity of UF fractions fractions (UF) from pepton. First day of crystallization at -4 °C.**

| | 1 | 2 | 3 | 4 |
|---|---|---|---|---|
| concentration added to wine (mg/l) | Peptone before fractionation | UF fraction (MW>10kDa) | UF fraction (3kDa<MW<10kDa) | UF fraction (MW<3kDa) |
| + 0 mg/l | 1 | 1 | 1 | 1 |
| +50 mg/l | 1 | precipitate | 1 | 1 |
| +100 mg/l | 1 | precipitate | 3 | 1 |
| +200 mg/l | 1 | precipitate | 6 | 1 |

### Example 6

One gram of gelatin (from pig skin, Sigma) was dissolved in 50 ml of tap water. The pH was adjusted to 8. Alcalase® (Sigma Aldrich) was added and incubation at 60 °C was done for 4 hours. The pH was readjusted to pH 8 every hour. After 4 hours no pH drop was observed, indicating that hydrolysis of protein had stopped.

The solution was fractionated by means of ultrafiltration (UF), first on a 10 kDa PM-10 polyethersulfon (PES) membrane (Amicon, Millipore, USA) in a stirred cell. Next, the permeate was fractionated on a 3 kDa YM-3 regenerated cellulose membrane (Amicon, Millipore, USA), yielding a retentate and a permeate. The retentate of the first ultrafiltration and the retentate and permeate of the second ultrafiltration were freeze-dried, and the total dry weight of the UF fractions relative to the initial gelatin starting material was calculated. The weight percentage of the several peptide fractions is given in table 12.

Most of the gelatin was hydrolyzed by Alcalase® in fragments smaller than 3kDa. However, a significant part was retained on the 3kDa membrane, and a very small part was also retained by a 10kDa membrane. Results are shown in table 13.

**Table 12: Total dry weight of UF fractions isolated from gelatine after treatment with protease (% w/w based on dry matter of starting material)**

| | MW >10 kDa | 3kDa<MW<10kDa | MW<3kDa |
|---|---|---|---|
| Yield | 1 | 19 | 80 |

**Table 13: Activity of UF fractions (UF) from hydrolyzed gelatin. First day of crystallization at -4 °C.**

| | 1 | 2 | 3 | 4 |
|---|---|---|---|---|
| concentration added to wine (mg/l) | Gelatin before protease | UF fraction (MW>10kDa) | UF fraction (3kDa<MW<10kDa) | UF fraction (MW<3kDa) |
| + 0 mg/l | 1 | 1 | 1 | 1 |
| +50 mg/l | precipitate | Missing* | <3 | <3 |
| +100 mg/l | precipitate | Missing* | 6 | <3 |
| +200 mg/l | precipitate | Missing* | 8 | <3 |

| | | | | |
|---|---|---|---|---|
| • not available due to low yield | | | | |

### Example 7

One gram of casein (Sigma) was dissolved in 50 ml of tap water. The pH was adjusted to 8. Alcalase® (Sigma Aldrich) was added and incubation at 60 °C was done for 4 hours. The pH was readjusted to pH 8 every hour. After 4 hours no pH drop was observed, indicating that hydrolysis of protein had stopped.

The solution was fractionated by means of ultrafiltration, first on a 10 kDa PM-10 polyethersulfon (PES) membrane (Amicon, Millipore, USA) in a stirred cell. Next, the permeate was fractionated on a 3 kDa YM-3 regenerated cellulose membrane (Amicon, Millipore, USA), yielding a retentate and a permeate.

The retentate of the first ultrafiltration and the retentate and permeate of the second ultrafiltration were freeze-dried, and the total dry weight of the UF fractions relative to the initial casein starting material was calculated. The weight percentage of the several UF fractions is given in table 14.

Most of the casein was hydrolyzed by Alcalase® in fragments smaller than 3kDa. However, a significant part was retained on the 3kDa membrane, and a very small part was also retained by a 10kDa membrane. Results are shown in table 15.

**Table 14: Total fry weight of UF fractions (UF) isolated from casein after treatment with protease (% w/w based on dry matter of starting material)**

| | MW >10 kDa | 3kDa<MW<10kDa | MW<3kDa |
|---|---|---|---|
| Yield | 0.2 | 26 | 74 |

**Table 15: Activity of UF fractions from hydrolyzed casein. First day of crystallization at -4 °C.**

| | 1 | 2 | 3 | 4 |
|---|---|---|---|---|
| Concentration added to wine (mg/l) | Casein before protease | UF fraction (MW>10kDa) | UF fraction (3kDa<MW<10kDa) | UF fraction (MW<3kDa) |
| + 0 mg/l | 1 | 1 | 1 | 1 |
| +50 mg/l | precipitate | Missing* | <3 | <3 |
| +100 mg/l | precipitate | Missing* | 5 | <3 |
| +200 mg/l | precipitate | Missing* | 7 | <3 |

| | | | | |
|---|---|---|---|---|
| not available due to low yield | | | | |

### Example 8

One gram of egg albumin (Sigma) was dissolved in 50 ml of tap water. The pH was adjusted to 8. Alcalase® (Sigma Aldrich) was added and incubation at 60 °C was done for 4 hours. The pH was readjusted to pH 8 every hour. After 4 hours no pH drop was observed, indicating that hydrolysis of protein had stopped.

The solution was fractionated by means of ultrafiltration, first on a 10 kDa PM-10 polyethersulfon (PES) membrane (Amicon, Millipore, USA) in a stirred cell. Next, the permeate was fractionated on a 3 kDa YM-3 regenerated cellulose membrane (Amicon, Millipore, USA), yielding a retentate and a permeate.

The retentate of the first ultrafiltration and the retentate and permeate of the second ultrafiltration were freeze-dried, and the total dry weight of the UF fractions relative to the initial egg albumin starting material was calculated. The weight percentage of the several peptide fractions is given in table 16.

Most of the egg albumin was hydrolyzed by Alcalase® in fragments smaller than 3kDa. However, a significant part was retained on the 3kDa membrane, and a very small part was also retained by a 10kDa membrane. Results are shown in table 17.

**Table 16: Total dry weight of UF fractions isolated from egg albumin after treatment with protease (% w/w based on dry matter of starting material)**

| | MW >10 kDa | 3kDa<MW<10kDa | MW<3kDa |
|---|---|---|---|
| Yield | 29 | 27 | 43 |

**Table 17: Activity of UF fractions from hydrolyzed egg albumin. First day of crystallization at -4 °C.**

| | 1 | 2 | 3 | 4 |
|---|---|---|---|---|
| Concentration added to wine (mg/l) | Egg albumin before protease | UF fraction (MW>10kDa) | UF fraction (3kDa<MW<10kDa) | UF fraction (MW<3kDa) |
| + 0 mg/l | 1 | 1 | 1 | 1 |
| +50 mg/l | precipitate | precipitate | 5 | <3 |
| +100 mg/l | precipitate | precipitate | 6 | <3 |
| +200 mg/l | precipitate | precipitate | 12 | <3 |

## Claims

1. The use of a composition in preventing and/or retarding the crystallisation of salts of tartaric acid in a wine, the composition comprising at least 2.5% of a peptide mixture based on dry weight of the composition, in which the peptides are from yeast and have a molecular weight between 3 kDa and 10 kDa.

2. A process to stabilise wine by preventing and/or retarding the crystallisation of salts of tartaric acid wherein a composition comprising at least 2.5% of a peptide mixture based on dry weight of the composition, **characterised in that** the peptides in the peptide mixture have a molecular weight between 3 kDa and 10 kDa is added to wine or to grape must to be used in the production of wine.

3. Process according to claim 2 wherein the composition is added to the wine or grape must to be used in fermentation for the production of wine in a concentration between 10-1000 mg of peptide mixture per litre of wine or must.

4. Process according to claim 2 or 3 wherein the wine is white wine or rosé wine.

5. A process according to any one of claim 2-4 wherein the composition is added to wine after filtration and before the bottling of the wine.

6. Process according to any one of claim 2-5 wherein the composition further comprises one or more biomolecules preferably selected from carbohydrates and/or oligonucleotides.

7. Process according to any one of claim 2-6 wherein the composition is in the form of a solid or in the form of a liquid or solution.

8. Process according to claim 7 wherein the composition is a liquid or solution and has a dry matter content between 50 - 500 g/l.

9. Process according to any of claims 2-8 wherein the composition further comprises one or more components, preferably selected from the group consisting of a protein substrate, substrate, a protein hydrolysate, a yeast extract, carboxymethyl cellulose and a wine additive.

10. Process according to claim 9, wherein the wine additive is mannoprotein.

11. Process according to claim 9, wherein the protein hydrolysate is of animal, vegetable or microbial origin.

## Patentansprüche

1. Verwendung einer Zusammensetzung für das Verhindern und/oder Hemmen der Kristallisation von Salzen von Weinsäure in einem Wein, wobei die Zusammensetzung bezogen auf das Trockengewicht der Zusammensetzung zumindest 2,5 % einer Peptidmischung umfasst, bei der die Peptide aus einer Hefe sind und ein Molekulargewicht zwischen 3 kDa und 10 kDa aufweisen.

2. Verfahren zum Stabilisieren von Wein durch Verhindern und/oder Hemmen der Kristallisation von Salzen von Weinsäure, wobei eine Zusammensetzung, welche bezogen auf das Trockengewicht der Zusammensetzung zumindest 2,5 % einer Peptidmischung umfasst, **dadurch gekennzeichnet, dass** die Peptide in der Peptidmischung ein Molekulargewicht zwischen 3 kDa und 10 kDa aufweisen, einem Wein oder einem bei der Produktion von Wein zu verwendendem Traubenmost hinzugefügt wird.

3. Verfahren nach Anspruch 2, bei dem die Zusammensetzung dem Wein oder dem bei der Fermentation für die Produktion von Wein zu verwendenden Traubenmost in einer Konzentration von 10 - 1000 mg Peptidmischung pro Liter Wein oder Most hinzugefügt wird.

4. Verfahren nach Anspruch 2 oder 3, bei dem der Wein Weißwein oder Rosewein ist.

5. Verfahren nach einem der Ansprüche 2 bis 4, bei dem die Zusammensetzung dem Wein nach Filtration und vor der Abfüllung des Weines hinzugefügt wird.

6. Verfahren nach einem der Ansprüche 2 bis 5, bei dem die Zusammensetzung weiter eine oder mehrere Biomoleküle umfasst, vorzugsweise ausgewählt aus Kohlenhydraten und/oder Oligonucleotiden.

7. Verfahren nach einem der Ansprüche 2 bis 6, bei dem die Zusammensetzung in Form eines Festkörpers oder in der Form einer Flüssigkeit oder Lösung vorliegt.

8. Verfahren nach Anspruch 7, bei dem die Zusammensetzung eine Flüssigkeit oder eine Lösung ist und einen Trockenmassegehalt zwischen 50 - 500 g/l aufweist.

9. Verfahren nach einem der Ansprüche 2 bis 8, bei dem die Zusammensetzung weiter eine oder mehrere Komponenten umfasst, vorzugsweise ausgewählt aus der Gruppe bestehend aus einem Proteinsubstrat, Substrat, einem Proteinhydrolysat, einem Hefeextrakt, Carboxymethylcellulose und einem Weinzusatz.

10. Verfahren nach Anspruch 9, bei dem der Weinzusatz ein Mannoprotein ist.

11. Verfahren nach Anspruch 9, bei dem das Proteinhydrolysat von einem Tier, Gemüse oder mikrobiellen Ursprungs ist.

## Revendications

1. Utilisation d'une composition pour prévenir et/ou retarder la cristallisation de sels d'acide tartrique dans le vin, la composition comprenant au moins 2,5% d'un mélange peptidique par rapport au poids sec de la composition, où les peptides sont issus de levure et ont un poids moléculaire compris entre 3 kDa et 10 kDa.

2. Procédé de stabilisation du vin en prévenant et/ou en retardant la cristallisation de sels d'acide tartrique, dans lequel une composition comprenant au moins 2,5% d'un mélange peptidique par rapport au poids sec de la composition, **caractérisé en ce que** les peptides dans le mélange peptidique ont un poids moléculaire compris entre 3 kDa et 10 kDa est ajoutée au vin ou au moût de raisin pour être utilisée dans la production de vin.

3. Procédé selon la revendication 2, dans lequel la composition est ajoutée au vin ou au moût de raisin pour être utilisée dans la fermentation pour la production de vin à une concentration comprise entre 10 et 1000 mg de mélange peptidique par litre de vin ou de moût.

4. Procédé selon la revendication 2 ou 3, dans lequel le vin est un vin blanc ou un vin rosé.

5. Procédé selon l'une quelconque des revendications 2 à 4, dans lequel la composition est ajoutée au vin après filtration et avant la mise en bouteilles du vin.

6. Procédé selon l'une quelconque des revendications 2 à 5, dans lequel la composition comprend en outre une ou plusieurs biomolécule(s) choisie(s) de préférence parmi des glucides et/ou des oligonucléotides.

7. Procédé selon l'une quelconque des revendications 2 à 6, dans lequel la composition se présente sous la forme d'un solide ou sous la forme d'un liquide ou d'une solution.

8. Procédé selon la revendication 7, dans lequel la composition est un liquide ou une solution et a une teneur en matière sèche comprise entre 50 et 500 g/l.

9. Procédé selon l'une des revendications 2 à 8, dans lequel la composition comprend en outre un ou plusieurs composant(s), choisi(s) de préférence dans le groupe constitué par un substrat protéique, un substrat, un hydrolysat protéique, un extrait de levure, la carboxyméthylcellulose et un additif pour le vin.

10. Procédé selon la revendication 9, dans lequel l'additif pour le vin est une mannoprotéine.

11. Procédé selon la revendication 9, dans lequel l'hydrolysat protéique est d'origine animale, végétale ou microbienne.
